# EUROPEAN PATENT SPECIFICATION

(11) **EP 1 490 096 B1**
(45) Date of publication and mention of the grant of the patent: **06.12.2006**
(21) Application number: 03745048.3
(22) Date of filing: 06.03.2003
(51) Int. Cl.: A61K 38/44, A61K 9/00, A61P 31/04

(54) **USE OF HYDROGEN PEROXIDE PRODUCING ENZYME FOR TREATMENT OF OTITIS MEDIA**
VERWENDUNG EINES WASSERSTOFFPEROXID PRODUZIERENDEN ENZYMS ZUR BEHANDLUNG VON OTITIS MEDIA
UTILISATION D'UNE ENZYME PRODUISANT DU PEROXYDE D'HYDROGENE POUR LE TRAITEMENT DE L'OTITE MOYENNE

(30) Priority: 22.03.2002 SE 0200876
(43) Date of publication of application: 29.12.2004
(73) Proprietor: TANO, Krister, 961 50 Boden (SE)
(72) Inventor: TANO, Krister, 961 50 Boden (SE)
(74) Representative: Lindgren, Anders
(86) International application number: PCT/SE2003/000384
(87) International publication number: WO 2003/080109

(56) References cited:
- EP-A1- 0 307 376
- EP-A2- 0 500 387
- US-A- 6 149 908
- US-B1- 6 214 339
- KRISTER TANO: 'Bacterial ecology of the nasopharynx in relation to otitis media' UMEA UNIVERSITY MEDICAL DISSERTATIONS, NEW SERIES no. 755, 2001, pages 1 - 63, XP002966587

## Description

### Field of the invention

The present invention relates to the use of hydrogen peroxide producing enzyme for manufacturing of a medicament for treatment and/or prevention of otitis media (inflammation in the middle ear), preferably in children.

### Background of the invention

Otitis media in children is the most common reason for antibiotic treatment of pre-school children. The present prophylactic treatment of choice, regarding children with recurrent otitis media, is placing tympanostomy tubes into the eardrums. This treatment has drawbacks as general anaesthesia, risk for persistent perforations in the eardrum, otorrhea and costs related to medical follow-up.
Long-term prophylaxis with antibiotics is a dominant treatment option in many countries. Drawbacks of this prophylactic treatment are a reduction of the normal nasopharyngeal bacterial flora, side effects of the antibiotics, and an increasing antibiotic resistance in the population.

Another way of preventing otitis media could be to apply bacteria taken from the normal bacterial flora, that possess a good inhibitory activity against otitis media pathogens, into the nostrils of otitis prone children. Previous studies have shown that the inhibitory activity of the alpha-haemolytic streptococci, AHS, (found in the normal bacterial flora) is reduced in children with recurrent episodes of otitis media, compared to healthy children [1]. The principle of "restoring" a normal bacterial flora with good inhibitory activity has been used in patients with recurrent tonsillitis and the results have indicated a reduction of tonsillitis episodes in those patients which used spray with AHS, compared to the patients in the placebo group [2]. Moreover, a nasal spray for children with recurrent otitis media, containing AHS or placebo, showed a significant reduction in otitis media episodes among the children using the spray with AHS [3]. Another placebo-controlled clinical study with a nasal spray containing AHS, showed no benefits compared to placebo [4]. The placebo in this study contained physiological saline and small amounts of skimmed milk. However, treatment comprising application of viable bacteria into the nose of children is not accepted in many countries.

It has been shown that one of the mechanisms used by AHS in order to inhibit growth of the otitis media pathogens, is to produce considerable amounts of hydrogen peroxide, H₂O₂ [5]. The enzyme NADH oxidase is most likely responsible for the AHS production of hydrogen peroxidase [6]. The hydrogen peroxide also contributes as a substrate for lactoperoxidase, LPO - a member of the non-specific defence system of the nasopharyngeal mucosa. Lactoperoxidase is dependent of hydrogen peroxide for production of hypothiocyanate, OSCN⁻, a more potent antibacterial substance. The normal bacterial flora including AHS and the human mucosa have a natural protection against both hydrogen peroxide and hypothiocyanate [7]. The synergistic effect between hydrogen peroxide and LPO is called the LPO system and is a part of the natural host defence system against invading micro organisms. It is commonly known that the concentration of hydrogen peroxide is a limiting factor for the antibacterial efficiency of the LPO system. The function of LPO is optimal when the concentration of hydrogen peroxide is about 1 mM [8]. AHS with good inhibitory activity is able to produce hydrogen peroxide to that concentration [9]. The LPO system has been used in a wide field of application which have resulted in granted patents (US6214339, US5503853, US5607681, US4578265 and US6149908). Different mixtures of peroxide producing enzymes and a suitable peroxidase in order to achieve the most potent antibacterial effect, have been developed (EP0500387, EP0307376 and US6312687).

Clinical studies have reported that Ringer's solution have a more beneficial effect on the mucociliary system of the nasal mucosa than physiological saline [10, 11]. This indicate that Ringer's solution is the preferable carrier solution for a pharmaceutical preparation compared to saline. Nasal irrigation with physiological saline is very important after nasal or sinus surgery in order to prevent infection and to restore the mucociliary function of the nasal mucosa. Thus, it would be more efficient to irrigate the nose with Ringer's solution than with physiological saline.

### Summary of the invention

Thus, one drawback of the prior art as earlier described is the use of live bacteria as the source of hydrogen peroxide for use in patients with otitis media. In the present invention it has surprisingly been found that it is possible to use hydrogen peroxide producing enzymes as an effective agent for treatment and/or prevention of otitis media.

Therefore, the invention as defined in claim 1, comprises the use of a hydrogen peroxide producing enzyme, selected from the group consisting of: glucose oxidase, NADH oxidase or amyloglucosidase in Ringer's solution for the manufacture of a medicament for treatment and/or prevention of otitis media.

### Brief description of the drawings

Figure 1.
   Trypsin added to PBS filtrate of alpha 89. The figure shows the means of two independent assays. Inter assay variation was within one log.
Figure 2.
   Catalase effect on the inhibitory activity of alpha 89. The figure shows the means of three independent assays. Inter assay variation was within one log.
Figure 3.
   Morphology of *H influenzae* after exposure of cell-free filtrate of alpha 89 for 6 hours, at 37°C.
Figure 4.
   Effect of amitrole on the inhibitory activity of alpha 4 on an isolate of *M. catarrhalis* in broth. The figure shows the means of two independent assays. Inter assay variation was within one log.
Figure 5.
   Hydrogen peroxide concentrations in cell-free filtrate of AHS and in fractions after gel filtration.

### Detailed description of the invention

The invention will now be described more closely in association with an experimental section.

The aim of the following description is to characterise the inhibitory substances from the isolates of AHS that had been the most effective inhibitors of three major otitis media pathogens.

As test bacteria clinical isolates of *H influenzae, S pneumoniae* and *M catarrhalis,* were used that were sampled from nasopharynx of patients with upper respiratory tract infections. It has earlier been shown that AHS isolates have good inhibitory activity against the test pathogens of *M catarrhalis, H influenzae* and *S pneumoniae.* Two of the isolates were chosen for more thorough studies regarding the mechanism of inhibition, the alpha 4 (*S. oralis*) and the alpha 89 (*S. sanguis*).

Numbers of colony forming units (CFU) were counted after serial dilutions in PBS and growth over night at 37°C in air. Identification of the bacteria was based on different morphology of the isolates growing on the agar plates. AHS and *M catarrhalis* were grown on agar plates with 7% human blood and McLeod plates were used when *H influenzae* was assayed.

To achieve a filtrate with inhibitory activity, about 10⁶ CFU of AHS were inoculated in 4 vials, each containing 15 ml of broth and incubated for 12 hours. The suspension was then centrifuged at 3000 rpm for 15 min and the supernatant was thrown away. The bacteria were transferred into one vial and two ml of phosphate buffered saline (PBS) was added and incubated at 37°C for 5 hours. The suspension was then passed through a 0.2 µm sterile filter to remove the bacteria. Otitis media pathogens (0.1ml) were then added to the filtrate (1 ml) and incubated at 37° C. The controls were incubated in fresh PBS.

Catalase is a specific enzyme catalyzing the conversion of H₂O₂ into O₂ and H₂O. This reaction proceeds rapidly. Purified catalase from human erythrocytes (>30.000U/mg) was used in the assays. The assays were performed as described above in the filtrate tests, with the exception that catalase, 1000 U/ml (=0.01ml), was added to the vials with filtrate and PBS. AHS filtrate in PBS without catalase was used as positive controls and fresh PBS with catalase was used as negative controls. Catalase was preincubated in the filtrate 10 min before the bacteria was added. The vials were incubated at 37°C in air.

In order to investigate if the inhibitory substance was a protein or a peptide, the filtrate was exposed to trypsin cutting peptide bonds. 2.5 mg of trypsin was weighted into test vials and 0.5 ml PBS filtrate of AHS was added. Samples were incubated at 37° C for 3 hours and thereafter boiled for 25 min. The suspension was allowed to cool before freezing (-20° C) until the next day. After thawing 10⁶ CFU/ml of *H. influenzae* was added. Finally, 150µl of broth was added and the suspension was allowed to incubate at 37° C.

Amitrole is an irreversible catalase inhibitor [12]. If an increased or more efficient catalase production was involved in the development of resistance of *M cararrhalis* to an isolate of AHS. This resistance would be reverted by addition of amitrole. In broth tests with alpha 4 together with *M catarrhalis* in 5 ml broth, 50 mM (0.1ml) of amitrole (3-amino-1,2,4-triazole), was added into the solution and incubated for 7 hours at 37°C in air with intermittent shaking. The isolate of *M catarrhalis* (Mcat-res) had developed resistance against alpha 4 after co-culturing in broth. CFU counts were calculated at start and after 7 hours of incubation. Alpha 4 with *M catarrhalis* in broth without amitrole, broth and *M catarrhalis* alone and *M catarrhalis* alone with amitrole in broth served as controls.

Size-exclusion chromatography was used as a step aiming to separate inhibitory substances found in the cell-free filtrates of AHS in PBS. By this procedure it is possible to separate substances in a solution according to their relative molecular weight. Cell-free filtrates were applied on a Superdex Peptide column (HR 10/30, Amersham Pharmacia Biotech, Sweden) equilibrated with 10 mM sodium phosphate, 0.5 M NaCl, pH=7.2. For each run, 250-500 µl sample was loaded on the column, the flow rate was 1 ml/min, and 1.5 ml fractions were collected. A mixture of proteins/peptides (50 µl) containing ribonuclease (13700 Da, 200 µ g/ml), bovine insulin B chain (3495 Da, 200 µg/ml), oxytocine (1007 Da, 200 µg/ml), glutathione (307 Da, 200 µg/ml) and glycine (75 Da, 7800 µg/ml) was applied on the column and elution volumes were used for calculation of relative molecular weights. A lower number of the fractions correspond to a higher relative molecule weight.

Ultracentrifugation gives a crude hint regarding the size of the inhibitory substances. Inhibitory substances consisting of protein molecules have a molecular weight above 30 kDa. Peptide bacteriocins (inhibitory substances) have a molecular weight between 5-10 kDa. Lanthionine containing bacteriocins (lantibiotics) typically lies between 1-5 kDa and small inorganic inhibitory substances have a molecular weight below 1 kDa.
Cell-free PBS filtrate of AHS were centrifuged step-wise through Microsep^{™} Microconcentrators (Filtron Technology Corporation) with molecule weight cut off (MWCO) at 30kDa, 10kDa, 5kDa and 1kDa. The centrifuging was done with 4000 rpm at 8°C. The centrifuged filtrate was assayed with the size-exclusion chromatography as described above and also tested for inhibitory activity with the filtrate method described above.

An assay for quantitative determination of H₂O₂ concentration of cell-free filtrate of AHS was performed as follows: A standard curve was first obtained by addition of known amounts of hydrogen peroxide to catalase treated and catalase inactivated cell-free filtrate of alpha 89 in PBS. 500µl Alfa-89a filtrate was treated with catalase (10000U/ml from human erythrocytes) for 15 minutes in 37°C before catalase inactivation for 35 minutes in 100°C. Thereafter, the filtrate was rationed out in portions of 50µl into Eppendorf tubes. 28µl of hydrogen peroxide, at different concentrations, was added to each tube to achieve final concentrations of 0 ; 1.0 ; 2.0 ; 3.0 ; 4.0 and 5.0 mM. These solutions served as standard points. 50µl from each tube and 50 µl test filtrates where respectively added to vials containing 200µl assay solution (200µl Phenol red (final conc. 0.2g/l), 200µl Horseradish peroxidase (final conc. 20U/ml), 9.6ml PBS). 100µl solution from each tube was added into wells on a micro titer plate. The plate was covered with a lid and incubated for 1.5h in 37°C. Finally, 10µl of 1M NaOH was added to all wells and after 5 minutes the colour was measured using a spectrophotometer (620nm).

PBS filtrate of alpha 4 and alpha 89 was produced according to the procedure above for electron microscopic examination. One ml of AHS filtrate was incubated together with 10⁷ CFU/ml of *M catarrhalis* or *H influenzae* (0.1 ml). Bacteria in fresh PBS were used as controls. CFU was counted at start and after 6 hours of incubation at 37° C in air. After 6 hours the vials with filtrate and bacteria were centrifuged at 3000 rpm for 15 min. The supernatant was removed and 3% glutaraldehyde was added. The pellets were incubated in the fixative for at least 24 hours. The specimens were then post-fixed in 1% osmium tetroxide in the same buffer, followed by dehydration in increasing concentrations of acetone and embedded in an epoxy resin. The plastic-embedded specimens were sectioned in 1µm thick sections with an ultra microtome and subsequently stained with toluidine blue. The specimens were then cut into ultra thin sections, 50-80 nm, and contrasted with uranyl citrate and lead citrate. The sections were examined in a JEM 1200 EX transmission electron microscope (TEM).

### Examples

The objective of the following examples are to further characterise the inhibitory substance released by the AHS, by using the alpha 4 and alpha 89 isolates as models.

### Example 1.

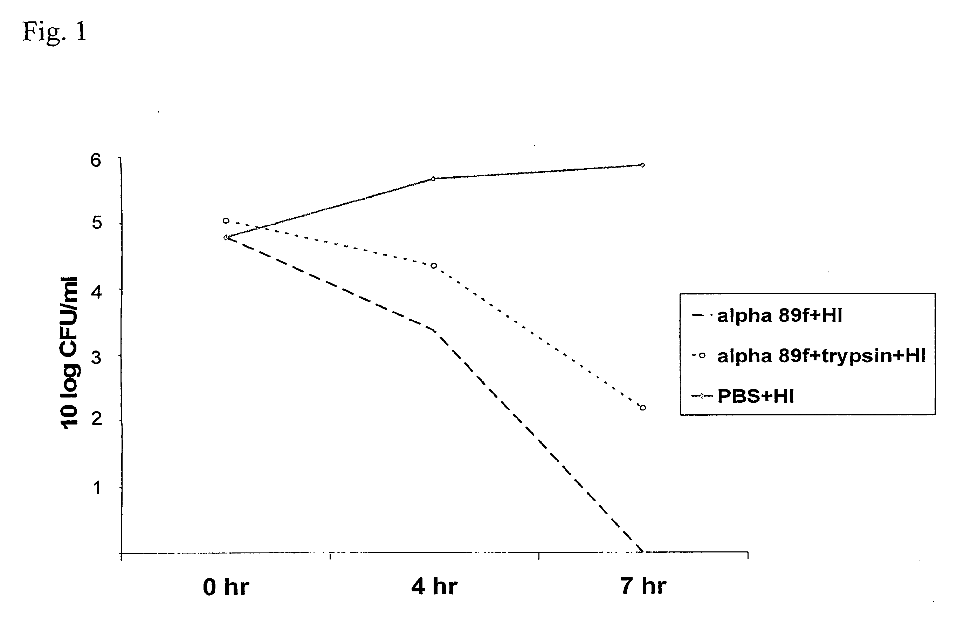

Trypsin (2.5mg/ml) could only slightly reverse the inhibitory effect of the cell-free filtrate of alpha 89 [fig 1]. Alpha 89 is an isolate of AHS with good inhibitory activity. HI is an isolate of *H influenzae.* Alpha 89f + HI = Cell-free filtrate of alpha 89 incubated together with an isolate of *H influenzae.* Trypsin together with PBS and *H influenzae* showed the same level of growth as PBS+HI
The results indicate that the inhibitory substance was not a protein or a peptide.

### Example 2.

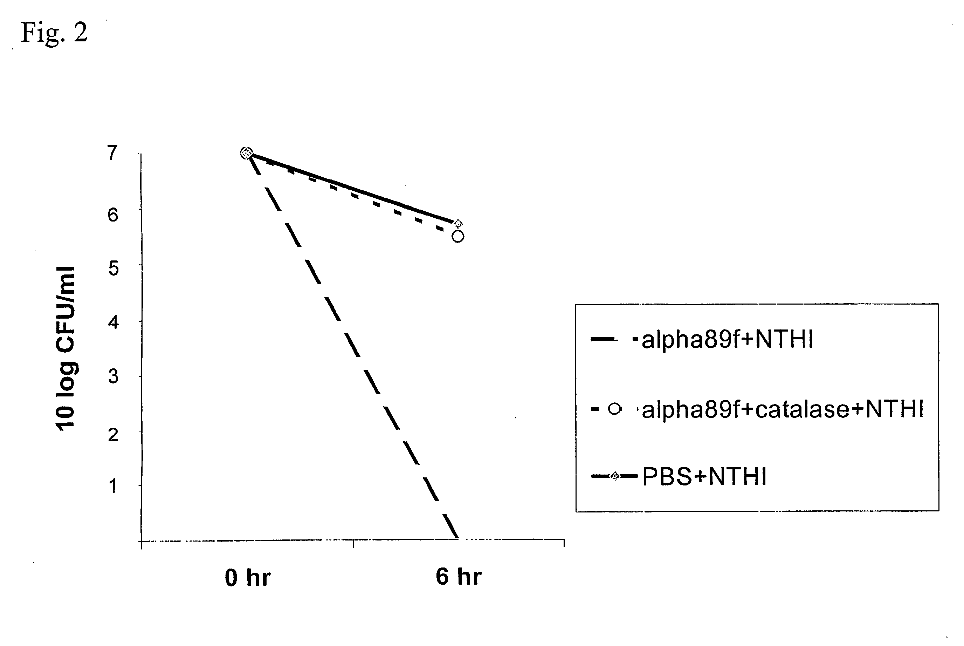

Catalase (1000U/ml) could completely reverse the inhibitory effect of the cell-free filtrate of AHS [fig 2]. Alpha 89f + HI = Cell-free filtrate of alpha 89 incubated together with the same isolate of *H influenzae* as in example 1. Catalase together with PBS and HI showed the same level as PBS+HI.
The results strongly indicate that hydrogen peroxide was the inhibitory substance.

The inhibitory activity of eight AHS isolates with a very good activity, tested with an agar overlay method, were also completely reversed with catalase.
Moreover, the inhibitory effect was not inactivated by boiling during 10 minutes. Eighteen hours in room temperature could inactivate the filtrate, but freezing at -80°C for 24 hours had no harmful effect on the inhibitory substance. The substance produced by alpha 4 and alpha 89 also seemed to be toxic against the bacteria itself in high concentrations. The inhibitory substances passed membranes of a MWCO of 1 kDa after ultra filtration and the chromatogram had the same appearance before and after passing through the filters. When fractions from the gel filtration were assayed regarding inhibitory activity, the inhibitory effect of the filtrate was found in the fractions corresponding to a molecular weight of less than 75 Da.

### Example 3.

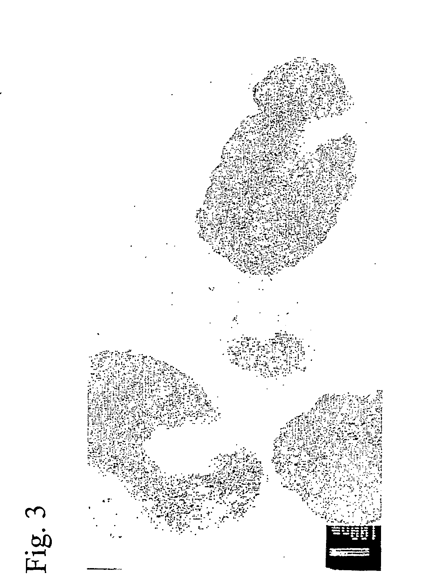

Morphology of *H influenzae* after exposure of cell-free filtrate of alpha 89 for 6 hours, at 37°C. The bacteria show pathologic changes with translucent and dense parts of the cytoplasm combined with bizarre cell membranes. These morphologic changes are similar to morphologic changes due to exposure of *H influenzae* to 5 mM of hydrogen peroxide for 6 hours.
Experiments with serially diluted hydrogen peroxide in PBS, assayed with *H influenzae* as in the filtrate tests, showed that the inhibitory effect of the AHS filtrate corresponded to a concentration of about 5mM (0.02%) hydrogen peroxide solution.
In light microscopy no morphological changes of the inhibited Gram stained bacteria were shown after 6 hours of incubation in AHS filtrate, in spite that the bacteria were not viable. In electron microscopy, the isolate of *H influenzae* that had been incubated with filtrate of alpha 89 or alpha 4 for 6 hours, showed disruptions of the cell wall membrane, a translucent protoplasm with dense parts and a bizarre cell configuration [fig 3].

### Example 4.

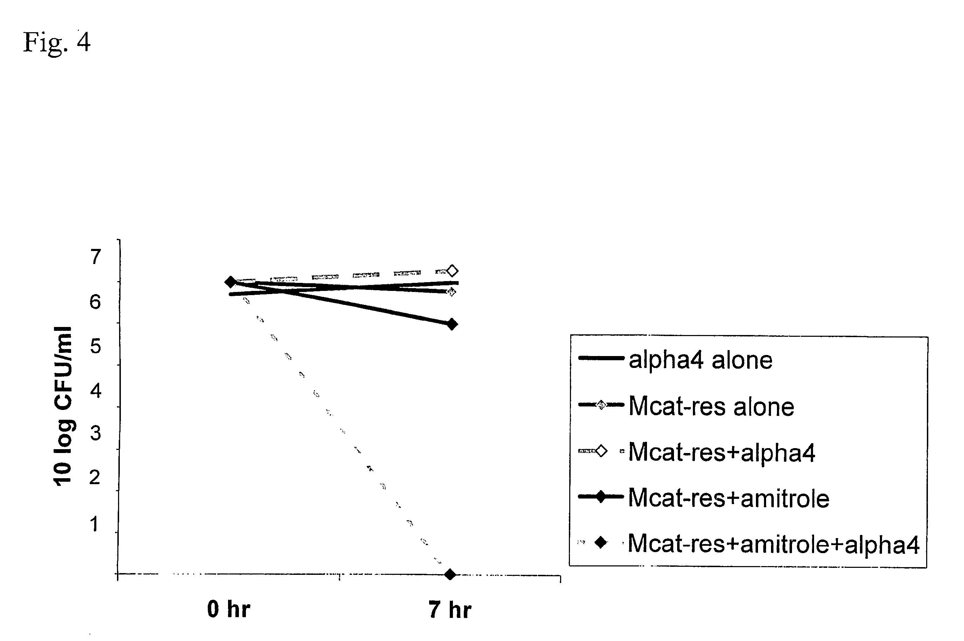

An isolate of *M catarrhalis* (Mcat-res), had been made resistant to the inhibitory effect of alpha 4 by co-cultivation in broth. In order to examine whether the catalase production of *M catarrhalis* could be responsible for this resistance, amitrole was added to the broth solution. When amitrole (50mM), an irreversible catalase inhibitor was added to the broth, the isolate of *M catarrhalis* became sensitive to the inhibition of alpha 4, thus indicating that an increased or more efficient catalase production was due to the resistance of *M catarrhalis* [fig 4]. Mcat-res+alpha 4= Growth of the resistant *M catarrhalis* together with alpha 4. Mcat-res + amitrole = Amitrole incubated together with the isolate of *M catarrhalis.* Alpha 4 together with amitrole or *M catarrhalis* showed the same growth as alpha 4 alone.
Adding amitrole to alpha 4 and the resistant isolate of *M catarrhalis* in broth showed that the resistance disappeared and the isolate of *M catarrhalis* became sensitive to inhibition by alpha 4.

### Example 5.

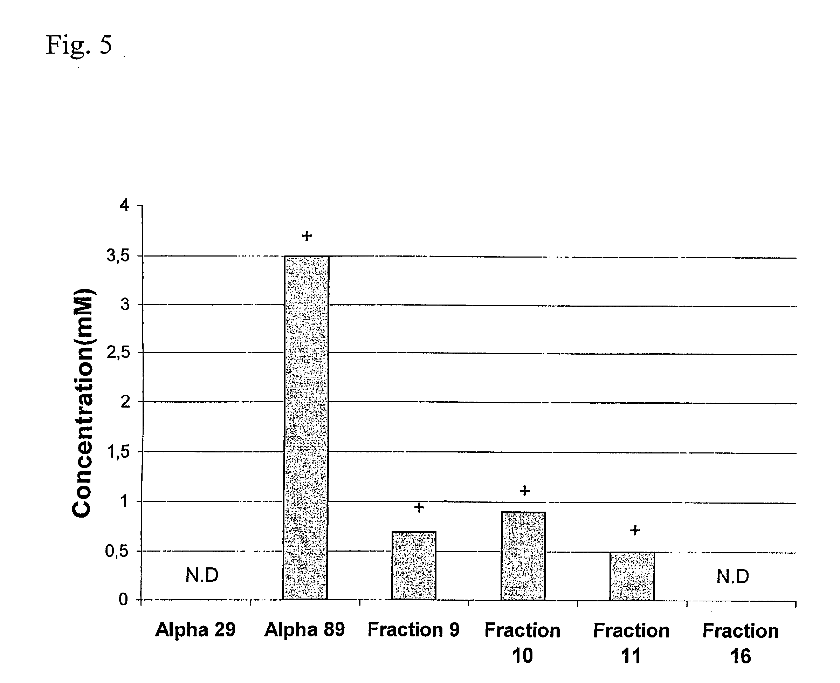

The quantitative assay showed a maximum hydrogen peroxide concentration of the cell-free filtrate of alpha 89 of 3.5 mM. Moreover, hydrogen peroxide was also detected in the gel filtration fractions with inhibitory activity [fig 5].

Quantitative determination of H₂O₂ -concentration in cell-free filtrate of AHS and in fractions of cell-free filtrate of alpha 89. Alpha 29 = Cell-free filtrate of alpha 29. Alpha 29 had a poor inhibitory activity on the otitis media pathogens in the agar overlay tests. Alpha 89 = Cell-free filtrate of alpha 89. Alpha 89 is an AHS with very good inhibitory activity on otitis media pathogens. N.D = Non-detectable. + = Inhibitory activity on *M catarrhalis.* The fractions tested represent samples from the size-exclusion chromatography. A lower number of the fractions correspond to a higher relative molecule weight. Fractions 9-11 corresponds to a molecule weight of less than 100 Da. Each of the fractions contained about 1.5 ml.

Trypsin treatment of the PBS filtrate of AHS could only slightly reverse the inhibitory effect, but the inhibitory effect of AHS completely disappeared when catalase was added. Catalase is an enzyme that converts hydrogen peroxide into H₂O and O₂. Moreover, amitrole, an irreversible catalase inhibitor, reversed the resistant isolate of *M catarrhalis* to an isolate sensitive to the inhibitory action by the AHS. After gel filtration inhibitory activity was only found in the fractions corresponding to very small molecular weights (75 Da). The morphologic examinations with TEM of the killed *H influenzae,* showed pathologic changes well correlated to damage caused by hydrogen peroxide [13,14]. A quantitative assay also showed high concentrations of hydrogen peroxide in the AHS filtrate, which correlates to the hydrogen peroxide levels found by Uehara et al [9]. Furthermore, only the fractions of AHS filtrate which contained substantial amounts of hydrogen peroxide showed inhibitory effect on *H influenzae.*
The findings above strongly suggest that the inhibitory effect of the AHS was related to their hydrogen peroxide production. The reason for the small reduction of the inhibitory activity of the filtrate found, when trypsin was added is not easy to explain, but the cell free filtrate of AHS probably contains extra cellularly released NADH-oxidase. NADH-oxidase is responsible for the hydrogen peroxide production of the AHS [6]. It is possible that the trypsin treatment of the filtrate inactivates the NADH-oxidase and thus reduces the production of hydrogen peroxide in the cell free filtrate of AHS. Trypsin had no catalase effect when tested together with hydrogen peroxide. The fact that no remaining inhibitory effect of the filtrate was observed after addition of catalase talks against a peptide bacteriocin. The short stability of the AHS filtrate in room temperature was probably due to the spontaneous oxidation of the hydrogen peroxide. Catalase production has shown to correlate to hydrogen peroxide resistance [15] of some bacteria and the relative resistance of *M. catarrhalis* to the inhibition of AHS could probably be ascribed to high levels of or a more efficient catalase production [16]. Amitrole is an irreversible catalase and lactoperoxidase inhibitor in the presence of hydrogen peroxide [12], and the presence of amitrole also increased the sensitivity of *M. catarrhalis* to inhibition by the AHS in the present study.

An ecological advantage of producing hydrogen peroxide, instead of producing a peptide bacteriocin, would be the broad spectrum of inhibitory activity of hydrogen peroxide; A bacteriocin produced by Gram positive bacteria is effective only against other Gram positive bacteria, while hydrogen peroxide is effective against both Gram positive and Gram negative bacteria, as well as against viruses and fungi.

The clinical significance of hydrogen peroxide production among the normal bacterial flora of nasopharynx could be very important since it opens new interesting interactions between the normal bacterial flora and the non-specific immune system. There are potential synergistic antimicrobial effects when combining hydrogen peroxide together with lactoperoxidase (LPO) and the non-specific immune system [17,18]. LPO converts thiocyanate (SCN⁻) produced by the salivary glands and hydrogen peroxide excreted from the normal bacterial flora into the more potent antibacterial substance, hypothiocyanate (OSCN⁻) [18]. AHS with good inhibitory activity, that is high hydrogen peroxide production, can provide LPO with the hydrogen peroxide needed. Hydrogen peroxide is thus directly or indirectly involved in the antibacterial effects of the normal flora (AHS), the non-specific immune system of the mucosa (LPO) and the polymorphonuclear granulocytes through their enzyme myeloperoxidase.

### REFERENCES

1. Tano K, Grahn Håkansson E, Holm SE, Hellström S; Inhibition of OM pathogens by alpha haemolytic streptococci from healthy children, children with SOM and children with rAOM. Int J Pediatr Otorhinolaryngol 2000;56:185-190
2. Roos K, Holm SE, Grahn E, Lind L; Alpha-Streptococci as Supplementary Treatment of Recurrent Streptococcal Tonsillitis: A randomized Placebo-controlled Study. Scand J Infect Dis 1993; 25:31-35
3. Roos K, Grahn-Håkansson E, Holm SE; Recolonization with interfering alpha-streptococci reduces the rate of recurrences in acute and secretory otitis media in otitis-prone children - a randomised placebo controlled study. BMJ 2001; 322: 210-212
4. Tano K, Grahn Håkansson E, Holm SE, Hellström S; A nasal spray with alpha-haemolytic streptococci as long term prophylaxis against recurrent otitis media. Int J Pediatr Otorhinolaryngol 2002; 62:17-23
5. Tano K; Bacterial ecology of the nasopharynx in relation to otitis media. Inhibitory activity and adherence of alpha-haemolytic streptococci in the prevention of otitis media in children. Umeå University medical dissertations, New series No 755, 2001 (study IV)
6. Carlsson J, Iwami Y, Yamada T; Hydrogen Peroxide Excretion by Oral Streptococci and Effect of Lactoperoxidase-Thiocyanate_Hydrogen Peroxide. Infect Immun 1983; 40: 70-80
7. Tenovuo J, Pruitt KM; Relationship of the Human Salivary Peroxidase System to Oral Health. J Oral Pathol 1984; 13:573-84
8. Pruitt KM, Tenuvuo JO; The Lactoperoxidase system-Chemistry and Biological significance. P.110-111. Marcel Dekker Inc New York 1985
9. Uehara Y, Kikuchi K, Nakamura T, Nakama H, Agematsu K, Kawakami Y, Maruchi N, Totsuka K; H₂O₂ Produced by Viridans Group Streptococci May Contribute to Inhibition of Methicillin-Resistant *Staphylococcus aureus* Colonization of Oral Cavities in Newborns. CID 2001; 32:1408-13
10. Unal M, Gorur K, Ozcan C; Ringer-Lactate solution versus Isotonic Saline solution on Mucociliary function after nasal septal surgery. J Laryngol Otol 2001; 115:796-7
11. Boek WM, Keles N, Graamans K, Huizing EH; Physiologic and hypertonic saline solutions impair ciliary activity *in vitro.* Laryngoscope 1999; 109: 3
12. Giulivi C, Hochstein P, Davies KJ; Hydrogen peroxide production by red blood cells. Free Radic Biol Med 1994; 16(1): 123-9
13. Silva MT; Electron microscopic study on the effect of the oxidation of ultrathin sections of Bacillus cereus and Bacillus megaterium. J Ultrastruct Res 1967; 18(3): 345-53
14. Rosan B, Eisenberg RJ; Morphological changes in Streptococcus sanguis associated with growth in the presence of oxygen. Arch Oral Biol 1973; 18(11): 1441-4
15. Ohwada T, Shirakawa Y, Kusumoto M, Masuda H, Sato T; Susceptibility to hydrogen peroxide and catalase activity of root nodule bacteria. Biosci Biotechnol Biochem 1999; 63(3): 457-62
16. Jouve HM, Lasauniere C, Pelmont J; Properties of a Catalase from a Peroxide-resistant Mutant of *Proteus mirabilis.* Can J Biochem Cell Biol 1983; 61: 1219-27
17. Thomas EL, Milligan TW, Joyner RE, Jefferson MM; Antibacterial activity of hydrogen peroxide and the lactoperoxidase - hydrogen peroxidase - thiocyanate system against oral streptococci. Infect Immun 1994; 62(2): 529-35
18. Ratner AJ, Prince A; Lactoperoxidase. New recognition of an "old" enzyme in airway defenses. Am J Respir Cell Mol Biol 2000; 22(6); 642-4

## Claims

1. Use of a hydrogen peroxide producing enzyme, selected from the group consisting of: glucose oxidase, NADH oxidase or amyloglucosidase in Ringer's solution for the manufacture of a medicament for treatment and/or prevention of otitis media.

2. Use according to claim 1 wherein any of said enzymes is combined with lactoperoxidase in said medicament.

3. Use according to any of the preceding claims wherein the medicament is water soluble.

4. Use according to any of the preceding claims wherein the medicament is formulated as a nasal spray.

5. Use according to any of claims 1-3 wherein the medicament is formulated as nose drops.

6. Use according to any of the previous claims wherein an enzyme substrate is added to the medicament.

## Patentansprüche

1. Verwendung eines Wasserstoffperoxid-produzierenden Enzyms ausgewählt aus der Gruppe bestehend aus Glucoseoxidase, NADH-Oxidase oder Amyloglucosidase in Ringerscher Lösung zur Herstellung eines Medikaments zur Behandlung und/oder Vorbeugung von Otitis media.

2. Die Verwendung gemäß Anspruch 1, worin irgendeines der Enzyme in dem Medikament mit Lactoperoxidase kombiniert wird.

3. Die Verwendung gemäß einem der vorangehenden Ansprüche, wobei das Medikament wasserlöslich ist.

4. Die Verwendung gemäß einem der vorangehenden Ansprüche, wobei das Medikament als Nasenspray formuliert ist.

5. Die Verwendung gemäß einem der Ansprüche 1 bis 3, wobei das Medikament als Nasentropfen formuliert ist.

6. Die Verwendung gemäß einem der vorangehenden Ansprüche, wobei dem Medikament ein Enzymsubstrat beigefügt ist.

## Revendications

1. Utilisation d'une enzyme produisant du peroxyde d'hydrogène, choisie dans le groupe constitué par : la glucose oxydase, la NADH oxydase ou l'amyloglucosidase dans une solution de Ringer pour la fabrication d'un médicament destiné au traitement et/ou à la prévention de l'otite moyenne.

2. Utilisation selon la revendication 1 dans laquelle l'une quelconque desdites enzymes est combinée à la lactoperoxydase dans ledit médicament.

3. Utilisation selon l'une quelconque des revendications précédentes dans laquelle le médicament est hydrosoluble.

4. Utilisation selon l'une quelconque des revendications précédentes dans laquelle le médicament est formulé sous la forme d'une pulvérisation nasale.

5. Utilisation selon l'une quelconque des revendications 1 à 3 dans laquelle le médicament est formulé sous la forme de gouttes nasales.

6. Utilisation selon l'une quelconque des revendications précédentes dans laquelle un substrat d'enzyme est ajouté au médicament.
